# EUROPEAN PATENT APPLICATION

(11) **EP 4 467 152 A1**
(43) Date of publication of application: **27.11.2024**
(21) Application number: 24176652.6
(22) Date of filing: 17.05.2024
(51) Int. Cl.: A61K 38/10, A61K 38/43, A61P 17/02, A61P 35/00, C12N 9/12

(54) **PEPTIDES FOR USE IN THE PREVENTION AND/OR TREATMENT OF RADIODERMATITIS**

(30) Priority: 22.05.2023 ES 202330394
(71) Applicant: Alodia Farmacéutica S.L.U., 28108 Alcobendas Madrid (ES)
(72) Inventor: Vázquez Rodríguez, Teresa, 15006 Coruña (ES); Ribagorda Muñoz, María, 05003 Ávila (ES)
(74) Representative: Pons

(57) **Abstract**

The present invention relates to a peptide characterized in that it comprises the sequence SEQ ID NO: 1, as well as other products derived thereof, for use in the treatment and/or prevention of radiodermitis in a subject, or in tissue regeneration following the application of radiotherapy to a subject. The products derived therefrom include the polynucleotide encoding the peptide and the vector comprising the peptide, as well as the cell, the nanoparticle and the composition comprising said peptide characterised in that it comprises the sequence SEQ ID NO: 1.

## Description

The present invention relates to a peptide characterized in that it comprises the sequence SEQ ID NO: 1, as well as other products derived thereof, for use in the treatment and/or prevention of radiodermitis in a subject, or in the regeneration of tissue following the application of radiotherapy to a subject. Therefore, the present invention falls within the field of medicine, more specifically, within the field of cancer treatment.

### STATE OF THE ART

One of the side effects of radiotherapy is epithelitis due to the effects of ionizing radiation, radiodermatitis. Greater knowledge of the factors that predispose to the appearance of radiodermitis and action on these factors could favour the prevention and delay in the development of this type of epithelitis. The role of nursing services is key to minimise the side effects of radiotherapy through the application of new treatments and their follow-up.

Acute radiodermatitis is caused by high-energy radiation, begins a few hours after exposure and its effects can last up to several weeks depending on the dose received; with accumulated doses of more than 7 Gy, clinical symptoms become evident. There are several classifications of the effects of acute and chronic radiodermatitis, with intermediate effects often overlapping. In general, chronic or late radiodermatitis is caused by exposure to high radiation (doses greater than 12-15 Gy) with skin lesions appearing weeks to years after radiation exposure. In this case, they do not depend so much on the speed of the radiation as on the accumulated dose.

In studies of radiodermatitis as a side effect of radiotherapy, the RTOG/ EORTC (Radiation Therapy Oncology Group/European Organization for Research and Treatment of Cancer) scale is frequently used. This scale allows the classification of the damage suffered by the skin of the radiated patient and the comparison between different radiotherapeutic treatments, moments of clinical evolution, palliative treatments, dermoprotectors. The RTOG/ EORTC scale defines the grades of epithelitis from grade 0 (G0) to grade 4 (G4). In the case of chronic radiodermatitis, the lesions and symptoms are not produced as an immediate effect of radiotherapy but appear months or even years after having received treatment with ionising radiation; in general, their appearance is associated with exposure to high doses of radiation.

Currently, treatment of acute radiodermatitis is based on the use of emollients and topical corticosteroids to relieve local symptoms. Occasionally, the use of indomethacin and oral non-steroidal anti-inflammatory drugs can help reduce pain and oedema. In chronic radiodermatitis lesions, healing is difficult as there is no granulation tissue, sometimes requiring surgical debridement and grafting of healthy skin. Hyperbaric Oxygen Therapy (HBO) is a non-invasive therapy, which is based on obtaining high oxygen partial pressures (two to three times higher than atmospheric pressure measured at sea level). The therapy is effective in a large number of pathologies, with an adequate cost/benefit ratio. In recent years, its use has been developed in the field of oncology, especially in the treatment and prevention of complications derived from the use of radiotherapy... However, these therapies have several disadvantages. On the one hand, moisturizing the skin with emollients is not enough in this type of lesions, as the skin has high levels of oxidation that must be repaired. On the other hand, the numerous side effects of corticotherapy are well known and can cause, among other things, skin and hair changes, weight gain and even systemic changes such as osteoporosis and blood pressure problems. Similarly, indomethacin and anti-inflammatory drugs also have frequent side effects, in this case related to digestive disturbances. Finally, HBO may be contraindicated in certain patients who have suffered from pathologies related to the ear or respiratory system, as well as in pregnant women and other population groups.

Therefore, there is a need in the state of the art to provide new therapies to treat the adverse skin effects of radiotherapy that do not have the disadvantages mentioned above.

### DESCRIPTION OF THE INVENTION

The inventors of the present invention have observed that peptides comprising the sequence SEQ ID NO: 1 have the ability to prevent and treat skin disorders in mammals and also in the mucous membranes of mammals. These alterations include irritation, desquamation, dehydration, dryness and hypersensitivity among others, all of them derived from the application of radiotherapy. In addition, they have also observed that these peptides have a broad tissue regenerative capacity.

These observations come from a clinical trial aimed at analyzing the effect of peptides comprising the sequence SEQ ID NO: 1 on the skin of patients who have received radiotherapy to treat breast and head-neck tumors, and comparing it with the usual moisturizing treatments. As can be seen in the Example of the present description, where the characteristics of the clinical trial are described in detail, the group of patients who had received treatment with the peptides comprising the sequence SEQ ID NO: 1 had a lower incidence of GI, GII and Gill degrees of epithelitis. In addition, these patients also showed that, when the dead skin was removed and cleaned, the new layer of skin was intact and the patient did not show the typical symptoms of stinging on contact with the open lesion.

Therefore, the use of peptides comprising the sequence SEQ ID NO: 1 in the treatment of adverse effects derived from radiotherapy opens a new therapeutic window for all those patients who receive this type of treatment. Based on this finding, a series of inventive aspects have been developed, which will be described in detail below.

*Peptide of the invention for use in the treatment and*/*or prevention of radiodermatitis, or in tissue regeneration following radiotherapy.*

In a first aspect, the present invention relates to a peptide characterized in that it comprises the sequence SEQ ID NO: 1, for use in the treatment and/or prevention of radiodermatitis, or in tissue regeneration following the application of radiotherapy.

### SEQ ID NO: 1: GFINLDKPSNP

Hereinafter, the peptide characterised in that it comprises the sequence SEQ ID NO: 1 will be referred to as "peptide of the invention".

As used in the present description, the term 'peptide' refers to a short sequence of amino acids, preferably from 2 to 100 amino acids (both ends included), linked by chemical bonds called peptide bonds (amide-type bond between the amino group (-NH2) of one amino acid and the carboxyl group (-COOH) of another amino acid).

The peptide of the invention can have any size between 11 and 100 amino acids (both ends included), provided that it comprises the sequence SEQ ID NO: 1. Thus, the size of the peptide of the invention can have a size between 11 and 20, 25, 30, 35, 40, 45, 50, 55, 60, 65, 70, 75, 80, 85, 90, 95, or 100 amino acids (both ends included).

In a particular embodiment of the peptide of the invention for use in the indicated treatment, alone or in combination with any and all of the above particular embodiments, the peptide comprises, or consists of, an amino acid sequence selected from the group consisting of SEQ ID NO: 2, SEQ ID NO: 3, SEQ ID NO: 4, SEQ ID NO: 5 and SEQ ID NO: 6.
SEQ ID NO: 2: GFINLDKPSNPSSHEVVAW
SEQ ID NO: 3: GFINLDKPSNPSSHEWAWIRRILR
SEQ ID NO: 4: GFINLDKPSNPSSHEWAWIRRILRVEKTGHSGTLDPKVTGCLIVCIERATRLVK
SEQ ID NO: 5: GFINLDKPSNPSSHEW
SEQ ID NO: 6: GFINLDKPSNPSSHEVVA

As the person skilled in the art understands, the peptide of the invention may have attached to its amino-terminal and/or carboxyl-terminal end other amino acid sequences which may be useful, for example, in the transport of the peptide into the cell interior, in the isolation of the peptide, in its identification, in its production, etc. The binding of the peptide of the invention to these other sequences can be direct or via a peptide linker or spacer between the two peptides. Any peptide with structural flexibility can be used as a peptide spacer; however, non-limiting illustrative examples of such peptide spacers include peptides containing amino acid moiety repeats, e.g. of Gly and/or Ser, or any other suitable amino acid moiety repeats.

Example amino acid sequences that can bind to the peptide of the invention include for the uses described above include, but are not limited to, a support peptide, a marker peptide, a signal peptide, such as a nuclear localization peptide.

In the context of the present invention, a "support peptide" is defined as a peptide with the ability to internalize a peptide into the cell. The supporting peptide is a peptide capable of crossing the cell membrane and entering the cell from the outside, a characteristic that can be conferred to the peptide (e.g. the peptide of the invention) with which it is fused, thus providing an alternative to the transport of the peptide of the invention into the target cells. This mechanism of peptide entry into the cell is known as "protein transduction or delivery". To implement the present invention, virtually any support peptide with the ability to internalize a peptide into a cell can be used; however, such a support peptide is a peptide comprising a "PTD" ("protein transduction domain") segment. Illustrative non-limiting examples of proteins comprising protein transduction domains (PTD) include the human immunodeficiency virus 1 (HIV-1) TAT protein, the Drosophila antennapedia homeotic transcription factor (Antp) and the herpes simplex virus 1 (HSV-1) DNA-binding protein VP22, although other proteins have also been suggested to have this property of internalizing peptides into cells, such as influenza virus haemagglutinin, lactoferrin, fibroblast growth factor 1, fibroblast growth factor 2 and the Hoxa-5, Hoxb-4 and Hoxc-8 proteins. Examples of supporting peptides include, but are not limited to:
- a peptide derived from the HIV-1 TAT protein, comprising the sequence responsible for peptide transduction, the basic domain (PTD) of which comprises moieties 49-57 of said HIV-1 TAT protein, specifically the amino acid sequence RKKRRQRRR (SEQ ID NO. 23), or moieties 47-57 of said HIV-1 TAT protein, such as the peptide whose amino acid sequence is YGRKRRQRRR (SEQ ID NO. 24): 23), or moieties 47-57 of said HIV-1 TAT protein, such as the peptide whose amino acid sequence is YGRKKRRQRRR (SEQ ID NO: 24) or the peptide whose amino acid sequence is CGISYGRKKKRRQRRR (SEQ ID NO: 25);
- a peptide derived from the Antp protein of D. melanogaster, comprising the antennapedia homeodomain (AntpHD) comprising the peptide transcription domain (PTD) [Fractions 43-58 of said Antp protein], comprising the amino acid sequence RQIKIWFQNRRMKWKKK (SEQ ID NO: 26), or a functional fragment thereof;
- a peptide derived from the VP22 protein of HSV-1 comprising a peptide transcription domain (PTD); and
- a peptide derived from the ARF tumor suppressor protein ('alternative reading frame') comprising the amino acid sequence responsible for the ability of the peptide to penetrate cells, such as the fragment comprising moieties 26-44 of said ARF protein, specifically, the amino acid sequence KFVRSRRRRPRTASCALAFVN (SEQ ID NO: 27), or a fragment thereof comprising fractions 37-44 of said ARF protein, specifically, the amino acid sequence SCALAFVN (SEQ ID NO: 28).

In the context of the present invention, a "marker peptide" is an amino acid sequence useful for the isolation or purification of the peptide of the invention. Such a sequence will be located in a region of the peptide of the invention that does not adversely influence the functionality of the peptide. Virtually any amino acid sequence that can be used to isolate or purify a fusion protein (generically referred to as tag peptides) can be present in the peptide of the invention. By way of non-limiting illustration, such amino acid sequence useful for isolation or purification of the peptide of the invention may be, for example, arginine tag (Arg-tag), histidine tag (His-tag), FLAG-tag, Strep-tag, an epitope that can be recognized by an antibody, such as c-myc-tag, SBP-tag, S-tag, calmodulin-binding peptide, cellulose-binding domain, chitin-binding domain, glutathione S-transferase-tag, maltose-binding protein, NusA, TrxA, DsbA, Avi-tag, etc. , β-galactosidase, VSV-glycoprotein (YTDIEMNRLGK) (SEQ ID NO: 29), or an amino acid sequence such as: AHGHRP (SEQ ID NO: 30) (2, 4 and 8 copies), PIHDHDHDHPHLVIHS (SEQ ID NO: 31), etc.

In the present invention "signal peptide" means a peptide present in a protein that can direct the protein towards the secretory pathway. A signal peptide is also known as a signal sequence, leader sequence or leader peptide. Typically, a signal peptide is a short peptide (e.g., 5-30, 5-25, 5-20, 5-15 or 5-10 amino acids in length). A signal peptide may be present at the N-terminal end of a newly synthesized protein. Examples of signal peptides include, but are not limited to, (i) nuclear localization signals - classical (NLS) , such as PKKKRKV (SEQ ID NO: 32), wherein the targeting compartment is the nucleus; (ii) Sec protein-dependent signal peptide, such as H2N-MDWTWRVFCLLAVTPGAHP (SEQ ID NO: 33), wherein the target compartment is the endoplasmic reticulum or outside the cell; or (iii) mitochondrial targeting signal; (iv) peroxisome targeting signal (PTS), such as PTS1 and PTS2, wherein the target compartment is the peroxisome.

Thus in one particular embodiment, alone or in combination with any and all of the above particular embodiments, the peptide of the invention comprises at least one nuclear localization sequence attached to at least one of the carboxyl or amino terminal ends of its amino acid sequence.

In yet another more particular embodiment, alone or in combination with any and all of the above particular embodiments, the nuclear localization sequence is attached to the carboxyl-terminal end of the peptide of the invention.

In the context of the present invention, "nuclear localization sequence" means the group of amino acids that directs the peptide to which they are bound to the nucleus of the cell; such a nuclear localization sequence can be either a transport sequence to the nucleus or a transport sequence to the nucleolus. Any nuclear localization sequence can bind to the peptide of the invention. However, in a particular embodiment, alone or in combination with any and all of the above particular embodiments, the nuclear localization sequence comprises the amino acid sequence SEQ ID NO: 7 or SEQ ID NO: 8.
SEQ ID NO: 7 (NLS1) KRKR
SEQ ID NO: 8 (NLS2) KKEKKKSK

In another more particular embodiment of the peptide of the invention, the peptide comprises, or consists of, the amino acid sequence SEQ ID NO: 9, SEQ ID NO: 10, SEQ ID NO: 11, SEQ ID NO: 12, SEQ ID NO: 13, SEQ ID NO: 14, SEQ ID NO: 15, or SEQ ID NO: 16.
SEQ ID NO: 9 GFINLDKPSNP**KRKR**
SEQ ID NO: 10 GFINLDKPSNPSSHEVVAWIRRILRVEKTGHSGTLDPKVTGCLIVCIERATRLVK**KRKR**
SEQ ID NO: 11 GFINLDKPSNPSSHEVVAWIRRILRVEKTGHSGTLDPKVTGCLIVCIERATRLVK**KKEKKKSK**
SEQ ID NO: 12 **KRKR**GFINLDKPSNPSSHEVVAWIRRILRVEKTGHSGTLDPKVTGCLIVCIERATRLVK
SEQ ID NO: 13 **KKEKKKSK**GFINLDKPSNPSSHEWAWIRRILRVEKTGHSGTLDPKVTGCLIVCIERATRLVK
SEQ ID NO: 14 **KRKR**GFINLDKPSNP
SEQ ID NO: 15 GFINLDKPSNP**KKEKKKSK**
SEQ ID NO: 16 **KKEKKKSK**GFINLDKPSNP

Shown in bold are the nuclear localization sequences attached to the amino- or carboxyl-terminal end of the peptide of the invention comprising the sequence SEQ ID NO: 1 (underlined).

The peptide of the invention can be obtained through the usual procedures known in the art for the synthesis of peptides: (i) by its chemical synthesis, or (ii) by inserting the nucleotide sequence encoding the peptide of the invention into expression vectors, and introducing said vector into a cell.

Peptide chemical synthesis procedures are widely known to the person skilled in the art, and include both solution or liquid phase synthesis and solid phase peptide synthesis (SPPS). Preferably, in the synthesis of the peptide of the invention, solid-phase peptide synthesis will be employed, using one of the methods known in the state of the art such as Fmoc and t-Boc.

Alternatively, the peptide of the invention can be synthesized by inserting the nucleotide encoding the peptide of the invention into an expression vector. Techniques, tools and methods for synthesizing the peptide of the invention are widely known in the prior art.

*Polynucleotide of the invention for use in the treatment and*/*or prevention of radiodermatitis, or in tissue regeneration following radiotherapy.*

In another aspect, the present invention relates to a polynucleotide encoding the peptide of the invention for use in the treatment and/or prevention of radiodermatitis in a subject, or in tissue regeneration following the application of radiotherapy to a subject.

In the present invention, "polynucleotide of the invention" means the nucleotide sequence whose expression in a cell gives rise to the peptide of the invention, i.e. the peptide comprising the sequence SEQ ID NO: 1.

As used in the present description, "polynucleotide" means a sequence of nucleotides covalently linked together to form a chain. The polynucleotide of the invention may be DNA, RNA or cDNA. The terms "polynucleotide", "nucleic acid" and "nucleic acid molecule" are used interchangeably to refer to polymeric forms of nucleotides of any length. Polynucleotides may contain deoxyribunucleotides, ribonucleotides, and/or their analogues. Nucleotides may have any three-dimensional structure, and may perform any function, known or unknown. The term "polynucleotide" includes, for example, single-stranded, double-stranded and triple helix molecules, a gene or gene fragment, exons, introns, mRNA, tRNA, rRNA, ribozymes, cDNA, recombinant polynucleotides, branched polynucleotides, plasmids, vectors, isolated DNA of any sequence, isolated RNA of any sequence, nucleic acid probes, and primers.

In a particular embodiment, alone or in combination with any or all of the above particular embodiments, the polynucleotide of the invention for use in the treatment and/or prevention of radiodermatitis in a subject, or in tissue regeneration following the delivery of radiotherapy to a subject, comprises the sequence SEQ ID NO: 17, SEQ ID NO: 18, SEQ ID NO: 19, SEQ ID NO: 20, SEQ ID NO: 21 or SEQ ID NO: 22.
SEQ ID NO: 17 ggtttcattaatcttgacaag ccc tctaac ccc
SEQ ID NO: 18 ggtttcattaatcttgacaag ccc tctaac ccc tcttcc cat gag gtg gtagcctgg
SEQ ID NO: 19 
ggtttcattaatcttgacaag ccc tctaac ccc tcttcc cat gag gtg
gtagcctggattcgacggatacttcgg
SEQ ID NO: 20 
ggtttcattaatcttgacaag ccc tctaac ccc tcttcc cat gag gtg
gtagcctggattcgacggatacttcgg gtg gag aag aca gggcacagt
ggt act ctg gat ccc aag gtg act ggttgtttaatc gtg tgcatagaacgagcc
act cgcttg gtg aag
SEQ ID NO: 21 ggtttcattaatcttgacaag ccc tctaac ccc tcttcc cat gag gtg gta
SEQ ID NO: 22 ggtttcattaatcttgacaag ccc tctaac ccc tcttcc cat gag gtg gtagcc

The polynucleotide of the invention for use in the treatment and/or prevention of radiodermatitis, or in tissue regeneration following the application of radiotherapy, may have attached to one or both of its 3' and/or 5' ends nucleotide sequences encoding a carrier peptide, a marker peptide, or a signal peptide such as a nuclear localisation peptide.

Thus, in one particular embodiment, alone or in combination with any or all of the above particular embodiments, the polynucleotide of the invention for use in the treatment and/or prevention of radiodermatitis, or in the regeneration of tissue following the application of radiotherapy, comprises attached to one end a nucleotide sequence encoding a nuclear localisation peptide.

As understood by the person skilled in the art, the polynucleotide of the invention can be inserted into a vector, primarily an expression vector, to introduce the polynucleotide of the invention into the cell. Thus, the present invention also relates to a vector comprising the polynucleotide of the invention for use in the treatment and/or prevention of radiodermatitis, or in the regeneration of tissue following the application of radiotherapy.

*Vector of the invention for use in the treatment and*/*or prevention of radiodermatitis, or in tissue regeneration following radiotherapy.*

In another aspect, the present invention relates to a vector comprising the polynucleotide of the invention, hereinafter "vector of the invention", for use in the treatment and/or prevention of radiodermatitis in a subject, or in the regeneration of tissue following the application of radiotherapy to a subject.

In a particular embodiment, the vector of the invention is an expression vector for use in the treatment and/or prevention of radiodermatitis in a subject, or in the regeneration of tissue following the application of radiotherapy to a subject.

The expression vector of the invention may comprise, in addition to the polynucleotide of the invention, a promoter directing its transcription (e.g. pT7, plac, ptrc, ptac, pBAD, ret, etc.), to which it is operatively linked, and other necessary or appropriate sequences controlling and regulating such transcription and, where appropriate, the translation of the product of interest (the peptide of the invention), e.g. transcription initiation and termination signals (tlt2, etc.), polyadenylation signal, origin of replication, ribosome binding sequences (RBS), sequences coding for transcriptional regulators (enhancers), silencers, ribosome binding sequences (RBS), transcriptional regulators (enhancers), silencers (RBS), silencers (RBS). ), polyadenylation signal, origin of replication, ribosome binding sequences (RBS), sequences encoding transcriptional regulators (enhancers), transcriptional silencers (silencers), repressors, etc. Examples of appropriate expression vectors can be selected according to the conditions and needs of each specific case from among expression plasmids, viral vectors (DNA or RNA), cosmids, artificial chromosomes, etc., which may also contain markers that can be used to select the cells transfected or transformed with the gene(s) of interest. The choice of vector will depend on the host cell and the type of intended use. Thus, in a particular embodiment of the vector of the invention, alone or in combination with any or all of the above particular embodiments, said vector is a plasmid or a viral vector. Such a vector can be obtained by conventional methods known to those skilled in the art.

Other elements that may comprise the vector of the invention are restriction enzyme recognition sequences and/or marker genes that allow the identification of cells that have incorporated the vector comprising the polynucleotide of the invention.

A "selection marker", "selection marker gene" or "indicator gene" includes any gene that confers a phenotype on a cell in which it is expressed to facilitate identification and/or selection of cells that are transfected or transformed with a vector as described herein. Suitable markers can be selected from markers that confer antibiotic resistance, that introduce a novel metabolic trait or that allow visual selection. Examples of selection marker genes include genes that confer antibiotic resistance (such as nptll which phosphorylates neomycin and kanamycin, or hpt, which phosphorylates hygromycin, or genes that confer resistance to, for example, bleomycin, streptomycin, tetracycline, chloramphenicol, ampicillin, gentamicin, geneticin (G418), spectinomycin or blasticidin), or genes that provide a metabolic trait. Expression of visual marker genes results in the formation of colour (e.g. 13-glucuronidase, GUS or 13-galactosidase with their colour substrates, e.g. X-Gal), luminescence (such as the luciferin/lucepharase system) or fluorescence (Green Fluorescent Protein, GFP, and derivatives thereof). This list represents only a small number of possible markers. The expert is familiar with these markers. Depending on the organism and the selection procedure, different markers are preferred. If desired, the genetic markers can be removed from the host cell at a later stage.

Once the vector of the invention has been defined, it has to be introduced into the host cell of choice.

Methods for introducing polynucleotides or vectors into a cell are widely known to the person skilled in the art and their use is standard laboratory practice. The term "introduce" in the context of the present invention refers to the incorporation of a nucleotide sequence into a cell by "transfection" or "transformation" or "transduction", where the nucleotide sequence may be incorporated into the genome of the cell (e.g. chromosome, plasmid, mitochondrial DNA), or be a stand-alone replicon, or be transiently expressed (e.g. transfected mRNA). As used herein, the terms "transformed", "stably transformed" or "transgenic", with reference to a cell, mean that the cell has a non-native (heterologous) nucleotide sequence integrated into its genome, or into an episomal plasmid that is maintained through multiple generations.

Examples of methods for introducing polynucleotides or vectors into host cells include, but are not limited to, electroporation; nuclear microinjection or direct microinjection into single cells; fusion of bacterial protoplasts with intact cells; use of polycations, e.g. polybrene or polyornithine; membrane fusion with liposomes, lipofectamine-mediated transfection, or lipofection; high-speed bombardment with DNA-coated microprojectiles; incubation with lipofectamine precipitation, or lipofection; membrane fusion with liposomes, lipofectamine-mediated transfection, or lipofection; high-speed bombardment with DNA-coated microprojectiles; incubation with DNA-calcium phosphate precipitation; DEAE-dextran-mediated transfection; infection with modified viral nucleic acids; Agrobacterium-mediated DNA transfer and the like.

Thus, in another aspect, the invention relates to a cell comprising the peptide of the invention, the polynucleotide of the invention, or the vector of the invention, for use in the treatment and/or prevention of radiodermitis, or in tissue regeneration following the application of radiotherapy.

Cell of the invention for use in the treatment and/or prevention of radiodermatitis, or in the regeneration of tissue following the application of radiotherapy.

In another aspect, the present invention relates to a cell comprising the peptide of the invention, the polynucleotide of the invention, or the vector of the invention, hereinafter "cell of the invention", for use in the treatment and/or prevention of radiodermitis in a subject, or in the regeneration of tissue following the application of radiotherapy to a subject. Transformed, transfected or infected cells can be obtained by conventional methods known to those skilled in the art, including, but not limited to, electroporation, protoplast fusion, co-precipitation with calcium phosphate (CaPO4) and precipitation with calcium chloride (CaCl2).

In principle, any cell can be used to incorporate the polynucleotide or vector of the invention. In a particular embodiment of the cell of the invention for use in the treatment and/or prevention of radiodermitis in a subject, or in tissue regeneration following the application of radiotherapy to a subject, the cell is a prokaryotic or eukaryotic cell.

Suitable prokaryotic host cells for the expression of the polypeptide of the invention include, but are not limited to, bacterial cells. Bacterial cells include, but are not limited to, Gram-positive bacterial cells such as species of the genus Bacillus, Streptomyces and Staphylococcus and Gram-negative bacterial cells such as cells of the genus Escherichia and Pseudomonas.

Eukaryotic host cells suitable for expression of the polypeptide of the invention include, but are not limited to, mammalian cells, plant cells, insect cells, and fungal cells. Fungal cells include, preferably, yeast cells such as Saccharomyces, Pichia pastoris and Hansenula polymorpha. Insect cells include, but are not limited to, Drosophila cells and Sf9 cells. Plant cells include, but are not limited to, cells from crop plants such as cereals, medicinal, ornamental or bulb plants. Mammalian cells suitable for use in the present invention include epithelial cell lines (porcine, human, etc.), osteosarcoma cell lines (human, etc.), neuroblastoma cell lines (human, etc.), epithelial carcinomas (human, etc.), glial cells (murine, human, etc.), hepatic cell lines (monkey, etc.), CHO cells (ChineseHuman, etc.), Drosophila cells (ChineseHuman, etc.), Drosophila cells (Drosophila, etc.), Sf9 cells (Drosophila, etc.) and Sf9 cells (Drosophila, etc.). ), CHO cells (ChineseHamsterOvary), COS cells, BHK cells, HeLa cells, 911, AT1080, A549, 293 or PER. C6, human ECCs 5 NTERA-2 cells, D3 cells of the mESCs line, human embryonic stem cells such as HS293 and BGV01, SHEF1, SHEF2 and HS181, NIH3T3, 293T, REH and MCF-7 cells and hMSCs (human mesenchymalstemcells), and GliNS2 cells (glioma stem cells).

In another more particular embodiment of the cell of the invention for use in the treatment and/or prevention of radiodermitis in a subject, or in the regeneration of tissue following the application of radiotherapy to a subject, the eukaryotic cell is a mammalian cell.

In another still more particular embodiment of the cell of the invention for use in the treatment and/or prevention of radiodermitis in a subject, or in the regeneration of tissue following the application of radiotherapy to a subject, the mammalian cell is a human cell.

*Nanoparticle of the invention for use in the treatment and*/*or prevention of radiodermatitis, or in tissue regeneration following radiotherapy.*

In another aspect, the present invention relates to a nanoparticle comprising the peptide of the invention, the polynucleotide of the invention, the vector of the invention or the cell of the invention, hereinafter "nanoparticle of the invention", for use in the treatment and/or prevention of radiodermatitis in a subject, or in tissue regeneration following the application of radiotherapy to a subject.

The term "nanoparticle" as used in the present invention refers to any material having dimensions in the range of 1-1,000 nm. In some embodiments, the nanoparticles have dimensions in the range of 2-200 nm, preferably in the range of 2-150 nm, and even more preferably in the range of 2-100 nm. The nanoparticles can contribute to preserving the integrity of the peptide, polynucleotide, vector, or cell of the invention until it reaches the target organ. In addition, the nanoparticles can also be modified to include moieties that allow the nanoparticle to be directed to an organ of interest. The nanoparticles that can be used in the context of the present invention can be formed of any biomaterial that allows maintaining the structure and functionality of the active ingredient (peptide, polynucleotide, vector or cell of the invention), and to be released in the target organ. Biomaterials most commonly used in the manufacture of nanoparticles are polymer composites such as alginate, chitosan, gelatin, hyaluronic acid, poly(lactic-co-glycolic acid) (PLGA), polylactide (PLA), polycaprolactone (PCL) and polyanionic cellulose (PAC). Their bioavailability, improved encapsulation, release control, and cyto- and biocompatible properties enhance the therapeutic value of the encapsulated agents.

In a particular embodiment of the nanoparticle of the invention for use in the treatment and/or prevention of radiodermitis in a subject, or in the regeneration of tissue following the application of radiotherapy to a subject, the nanoparticle is a nanosphere (spherical shaped nanoparticle), a nanocapsule, a micelle, a liposome or a dendrimer.

In the present invention, the term "nanospheres" refers to a spherical matrix system, which have the active agent homogeneously dispersed in a polymeric matrix.

In the present invention, the term "nanocapsules" refers to the vesicular system surrounding the active agent within a cavity surrounded by a polymeric shell, which controls its release depending on its nature.

In the present invention, the term "micelle" refers to the conglomerate of molecules which constitutes one of the colloid phases, and within which the active substance is encapsulated.

In the present invention, the term "liposome" refers to a spherical vesicle (small bubble) with a membrane composed of a phospholipid bilayer, consisting of water-soluble and fat-soluble parts.

In the present invention, the term "dendrimer" refers to a three-dimensional molecule of arborescent construction and polymeric nature, wherein the distribution of molecules constituting linear polymers have a chemical structure exhibiting a generational growth form, G0, G1, G2.

The materials and methods for the manufacture of nanoparticles and the encapsulation of active ingredients within them are widely known in the state of the art and are routine practice for the person skilled in the art.

*Composition of the invention for use in the treatment and*/*or prevention of radiodermatitis, or in tissue regeneration following radiotherapy.*

In another aspect, the present invention relates to a composition comprising the peptide of the invention, the polynucleotide of the invention, the vector of the invention, the cell of the invention, or the nanoparticle of the invention, hereinafter "composition of the invention", for use in the treatment and/or prevention of radiodermatitis in a subject, or in tissue regeneration following the application of radiotherapy to a subject.

In a particular embodiment of the composition of the invention for use in the treatment and/or prevention of radiodermitis in a subject, or in the regeneration of tissue following the application of radiotherapy to a subject, the composition comprises a vehicle and/or an excipient.

The term "vehicle" refers to a diluent with which the active substance is administered. Such vehicles may be sterile liquids, such as water and oils, including those of petroleum, animal, vegetable or synthetic origin, such as peanut oil, soybean oil, mineral oil, sesame oil and the like. Water or aqueous solutions of saline and aqueous solutions of dextrose and glycerol are preferably used as vehicles, particularly for injectable solutions.

The term "excipient", as used herein, refers to an inert substance that is commonly used as a diluent, preservative, binder, or stabilizing agent for drugs that confer a beneficial physical property to a formulation, such as increasing protein stability, increasing protein solubility, and decreasing viscosity. Examples of excipients include, but are not limited to, proteins (e.g., but not limited to, serum albumin), amino acids (e.g., but not limited to, aspartic acid, glutamic acid, lysine, arginine, glycine), surfactants (e.g., but not limited to, SDS, Tween 20, Tween 80, polysorbate and non-ionic surfactants), saccharides (e.g., but not limited to, glucose, sucrose, maltose and trehalose), polyols (e.g., but not limited to, mannitol and sorbitol), fatty acids and phospholipids (e.g., but not limited to, sulfonates and alkyl caprylate).

In a particular embodiment, alone or in combination with the other particular embodiments, the composition of the invention is a pharmaceutical composition.

In the present invention, "pharmaceutical composition" means any pharmaceutical preparation or form, the formula of which, expressed in units of the international system, consists of a substance or mixture of substances, with constant weight, volume and percentages, manufactured in legally established pharmaceutical laboratories, packaged or labelled for distribution and marketed as effective for the diagnosis, treatment, mitigation and prophylaxis of a disease, physical anomaly or symptom, or the restoration, correction or modification of the balance of the organic functions of human beings and animals. The preparation of the pharmaceutical composition can be carried out by any of the methods described in the prior art.

Thus, in a more particular embodiment of the composition of the invention for use in the treatment and/or prevention of radiodermitis in a subject, or in the regeneration of tissue following the application of radiotherapy to a subject, the vehicle and/or excipient are pharmaceutically acceptable. The phrase "pharmaceutically acceptable" as used herein means approved by a federal or state government regulatory agency, or listed in the United States Pharmacopoeia, European Pharmacopoeia or other generally recognized pharmacopoeia for use in animals, and more particularly in humans.

Thus, in the case of pharmaceutical vehicles, these are either approved by a state or federal government regulatory agency or are listed in the US Pharmacopoeia or other generally recognized pharmacopoeia for use in animals, and more particularly in humans. The vehicles and auxiliary substances necessary to manufacture the desired pharmaceutical dosage form of administration of the pharmaceutical composition of the invention will depend, among other factors, on the chosen pharmaceutical dosage form of administration. Such pharmaceutical dosage forms for administration of the pharmaceutical composition will be manufactured according to conventional methods known to the person skilled in the art.

The peptide, polynucleotide, vector, cell, or nanoparticle of the invention may be in a therapeutically effective amount. In the present invention, "therapeutically effective amount" means the amount of active substance that provides some improvement or benefit to the subject. In other words, a "therapeutically effective" amount is an amount that promotes some relief, mitigation and/or reduction of at least one clinical symptom. Additionally, those skilled in the art will appreciate that the therapeutic effects need not be complete or curative, as long as some benefit is provided to the subject.

The appropriate dosage of the active ingredient(s) within the composition of the invention will depend on the type of tissue damage to be treated, the severity and course of the disease or damage, whether the composition is administered for preventive or therapeutic purposes, prior therapy, the patient's medical history and response to the peptide or polypeptide, and the discretion of the treating physician. The amount of peptide, polynucleotide, cell or nanoparticle of the invention is appropriately administered to the patient or subject at one time or during a series of treatments. The pharmaceutical or cosmetic composition can be administered for at least 1, 2, 3, 4, 5, 6, 7, 14, 21, 28 or more days. The pharmaceutical or cosmetic composition may preferably be administered 2 to 3 times a day for the required period of time.

The composition of the invention can be applied to areas of the body requiring treatment by subcutaneous injection, intradermal injection, and occlusive cure or by iontophoresis, in order to achieve greater penetration of the active principle.

In another particular embodiment of the composition of the invention for use in the treatment and/or prevention of radiodermitis in a subject, or in the regeneration of tissue following the application of radiotherapy to a subject, the composition is formulated for topical or cutaneous application to the subject.

In the present invention "topical application" is understood to mean application to the exterior of the body, such as, for example, the skin, scalp and nails; and also application to mucous membranes such as, without limitation, oral, nasal, rectal, vaginal and ocular mucosa. Thus, for this purpose, the composition of the invention may be presented, for example and without limitation, in the form of an aqueous, hydroalcoholic or oily solution, an oil-in-water or water-in-oil emulsion, or multiple, of an aqueous or oily gel, of a liquid anhydrous product, of an oil dispersion in an aqueous phase with the aid of spheroids (such as for example nanospheres, nanocapsules and vesicles), nanocapsules and lipid vesicles), silicone emulsions in water, microemulsions, pastes, milks, balms, foams, lotions, creams, soaps, hydroalcoholic solutions, hydroglycolic solutions, liniments, serums, serums, polysaccharide films, ointments, fatty ointments, mousses, ointments, gels, cream gels, foam gels, emulsion gels and solutions.

On the other hand, topical administration of the composition of the invention can also be performed through materials comprising or coated with the peptide, composition, cell, nanoparticle, or composition of the invention. Illustrative, non-limiting examples of such materials include bandages, gauze, or skin grafts.

The composition of the invention may contain additional ingredients commonly employed in skin care and treatment compositions. These additional ingredients include, but are not limited to, emulsifying agents, emollients, organic solvents, skin conditioners such as humectants, alpha hydroxy-acids, moisturizers, vitamins, pigments or dyes, gelling polymers, thickeners, softeners, anti-wrinkle agents, agents capable of reducing or treating bags under the eyes, bleaching or depigmenting agents, exfoliating agents, anti-ageing agents, free radical scavengers and/or anti-atmospheric pollution agents, NO-synthase inhibitors, antioxidant agents, anti-glycation agents, agents stimulating the synthesis of dermal or epidermal macromolecules and/or capable of inhibiting their degradation, such as collagen synthesis stimulating agents, elastin synthesis stimulating agents, laminin synthesis stimulating agents, decorin synthesis stimulating agents, collagen degradation inhibiting agents, elastin degradation inhibiting agents, fibroblast proliferation stimulating agents, keratinocyte proliferation stimulating agents, keratinocyte differentiation stimulating agents, lipid and stratum corneum component synthesis stimulating agents (ceramides, fatty acids, etc.), dermo-relaxing agents, skin softening agents, keratinocyte differentiation stimulating agents, keratinocyte differentiation stimulating agents, lipid and stratum corneum component synthesis stimulating agents (ceramides, fatty acids, etc.), dermo-relaxing agents. ), dermo-relaxing agents, glycosaminoglycan synthesis stimulating agents, firming agents, anti-stretch mark agents, soothing agents, anti-inflammatory agents, agents acting on capillary circulation and/or microcirculation, agents acting on cell metabolism, agents stimulating and/or inhibiting melanin synthesis, agents intended to improve the dermis-epidermis junction, antimicrobial agents, preservatives, perfumes, chelating agents, plant extracts, essential oils, marine extracts, bio-fermentation agents, mineral salts, cellular extracts and sunscreens (photoprotective agents of organic nature or mineral nature active against ultraviolet rays A and B) among others, provided that they are physically and chemically compatible with the other components of the composition and in particular with the peptides of general formula (I) of the present invention. The nature of such additional ingredients may be synthetic or of natural origin, such as plant extracts. Furthermore, the composition of the invention may contain or may be co-administered with analgesic and/or anti-inflammatory compounds in order to reduce swelling and irritation. Such compounds may include steroidal compounds such as hydrocortisone, non-steroidal compounds such as paracetamol or acetylsalicylic acid, or natural extracts or essential oils with intrinsic analgesic and anti-inflammatory activity.

*Kit of the invention for use in the treatment and*/*or prevention of radiodermatitis, or in tissue regeneration following radiotherapy.*

As understood by the person skilled in the art, the peptide, polynucleotide, vector, cell, nanoparticle or composition of the invention may be part of a kit.

Thus, in another aspect, the present invention relates to a kit comprising the peptide, polynucleotide, vector, cell, nanoparticle or composition of the invention, hereinafter kit of the invention, for use in the treatment and/or prevention of radiodermatitis in a subject, or in the regeneration of tissue following the application of radiotherapy to a subject.

In the present invention, "kit" means a product containing the different components or active ingredients necessary to implement the invention, and optionally, the clinical material necessary for its proper administration to the subject to be treated.

Components useful for administration and which may be included in the kit include, but are not limited to, buffer solution, lysis solution, sterile material (such as syringes, swabs, swabs or forceps), distilled water or alcohols (ethanol). Additionally, the kit may contain instructions or directions to guide the person skilled in the art in its administration.

Either the peptide, polynucleotide, vector, cell, nanoparticle of the invention or the composition of the invention are useful for use in the treatment and/or prevention of radiodermatitis, or in the regeneration of tissue following the application of radiotherapy.

In the present invention, "treating" or "treating" means that the severity of the subject's condition (in the present invention, radiodermitis) is reduced or at least partially improved or alleviated and/or that some relief, mitigation or decrease in at least one clinical symptom is achieved and/or there is an inhibition or delay in the progression of the condition and/or prevention or delay in the onset of a disease or illness. Therefore, the terms "treat", "treating" or "treatment of" (or grammatically equivalent terms) refer to both prophylactic and therapeutic treatment regimens.

On the other hand, as used herein, the terms "prevent", "preventing" and "prevention" refer to the inhibition of the development or occurrence of a disease or disorder (in the present invention radiodermitis), or the prevention of recurrence, occurrence or development of one or more symptoms of a disease or disorder in a subject resulting from the administration of a therapy (in the present invention the peptide, polynucleotide, vector, cell, nanoparticle of the invention or composition of the invention), or the administration of a combination of therapies (e.g., a combination of prophylactic or therapeutic agents).

The disease or disorder to be treated and/or prevented in the present invention is radiodermatitis. Radiodermatitis" refers to the group of skin lesions that appear after exposure of the skin to ionizing radiation. These exposures can be either therapeutic or accidental. These changes depend on the total dose received, the depth of penetration of the radiation and individual sensitivity. Examples of changes include, but are not limited to, early transient erythema, erythema, pruritus, epilation, dry desquamation, wet desquamation, hyperpigmentation, ulceration, dermal necrosis, fibrosis, and telangiectasia.

Thus, in a particular embodiment of the peptide, polynucleotide, vector, cell, nanoparticle or composition of the invention for use in the treatment and/or prevention of radiodermitis in a subject, radiodermitis comprises the occurrence of skin desquamations, skin erosions, skin and mucous membrane irritations, and/or skin and mucous membrane dehydration.

Depending on the time of onset of the lesions, a distinction is made between an acute and a chronic form. In the present invention, both types of forms can be treated and/or prevented by administering the polynucleotide, vector, cell, nanoparticle and composition of the present invention to the subject.

On the other hand, the peptide such as polynucleotide, vector, cell, nanoparticle and composition of the invention can also be used for tissue regeneration following radiotherapy to a subject. In the present invention, "tissue regeneration" means the process by which the structure and function of damaged organs or body parts are restored.

Likewise, any subject who is to receive radiotherapy and is susceptible to radiodermatitis can be treated with the peptide, polynucleotide, vector, cell, nanoparticle or composition of the invention. In the present invention the terms "subject", "individual" and "patient" are equivalent and may be used interchangeably. As used in this description, the term "subject" refers to any animal, preferably a mammal, more preferably a primate, in particular, a human being, of any sex or age.

Thus, in a particular embodiment of the peptide, polynucleotide, vector, cell, nanoparticle or composition of the invention for use in the treatment and/or prevention of radiodermitis in a subject, or in the regeneration of tissue following the application of radiotherapy to a subject, the subject is a human being.

Equivalently, to the use described above, the present invention also contemplates the use of the peptide, polynucleotide, vector, cell, nanoparticle or composition of the invention, in the preparation of a medicament for the treatment and/or prevention of radiodermatitis in a subject, or for the regeneration of tissue following the application of radiotherapy to a subject. All the particular embodiments described above for the various inventive aspects, as well as definitions and explanations, are applicable to the present use.

Finally, the present invention also relates to a method for the treatment and/or prevention of radiodermitis in a subject, or for tissue regeneration following the application of radiotherapy to a subject, comprising administering to said subject the peptide, polynucleotide, vector, cell, nanoparticle or composition of the invention. Similarly, all the particular embodiments described above for the various inventive aspects, as well as definitions and explanations, are applicable to the present method.

### DESCRIPTION OF THE FIGURES

**Figure 1****.** Percentage of patients in breast localization field who do not present any degree of epithelitis 10 days after completion of radiotherapeutic treatment in the control treatment groups versus the treatment group with the composition of the invention.
**Figure 2****.** Radiodermatitis grades 10 days after completion of radiotherapy treatment in the control treatment groups versus the treatment group with the composition of the invention in breast localization.
**Figure 3****.** Percentage of patients in the head-neck localization field who do not present any degree of epithelitis 10 days after completion of radiotherapeutic treatment in the control treatment groups versus the treatment group with the composition of the invention.
**Figure 4****.** Degrees of radiodermatitis 10 days after completion of radiotherapy treatment in the control treatment groups versus the treatment group with the composition of the invention in head-neck location.
**Figure 5****.** Grades of post-radiotherapy epithelitis in a sample of 169 patients according to the field of application (breast, head-neck, rectal/anal or vulva/gynecological).
**Figure 6****.** Percentage of patients in breast localization field who do not present any degree of epithelitis 10 days after completion of radiotherapy treatment in the control treatment groups versus the treatment group with the composition of the invention.
**Figure 7****.** Percentage of patients in the head-neck localization field who do not present any degree of epithelitis 10 days after completion of radiotherapy treatment in the control treatment groups versus the treatment group with the composition of the invention.
**Figure 8****.** Percentage of patients developing epithelitis (any grade) in the breast and head-neck localization fields in the control treatment groups versus the treatment group with the composition of the invention.

### EXAMPLE

In the following, the invention will be illustrated by means of tests carried out by the inventors, which demonstrate the effectiveness of the product of the invention.

### Effect of the application of a composition comprising the peptide of the invention on the evolution of radiodermatitis in patients treated with radiotherapy.

### MATERIALAND METHODS

### Treatment

Patients subjected to this trial were administered a composition in which a nutrient-based moisturizing base, a growth factor involved in connective tissue restructuring and the peptide comprising the sequence GFINLDKPSNP (SEQ ID NO: 1) are conjugated. Hereinafter referred to as the composition of the invention.

### Study subjects

An observational, longitudinal and prospective study was conducted between October 2013 and March 2014 with 56 patients indicated for breast and head-neck radiotherapy. These patients were randomly divided into two groups: group I, consisting of 19 patients (14 breast and 5 head-neck patients), received a first application of the composition of the invention by means of a gentle massage on the area affected by the radiation and were instructed to repeat the application twice a day. The control group, consisting of 36 patients (26 with breast cancer and 10 with head-neck cancer), were treated with the usual moisturizer (Gold Standard: comprising emollients, moisturizers, preservatives and perfumes) used in the nursing office of the Hospital Clinico San Carlos in Madrid.

Both groups had similar characteristics in terms of age and dose of radiotherapy received. It is important to bear in mind that the toxic effect of radiotherapy is cumulative as a function of the total dose received. Thus, the toxicity of a radiotherapy session is not only determined by the daily dose received but also by the total cumulative dose. The most frequent Gy/day dose observed in the patients analyzed was 2 Gy per day and the mean cumulative total dose at the end of treatment was 56 Gy.

From the day of planning the radiotherapy sessions and throughout the treatment, the patients were informed and advised on the preventive care to be applied according to the degree of epithelitis, which is summarized in table 1.

The collection of data on the evolution of the epithelial lesions generated a register adjusted to equivalent criteria carried out weekly and using the RTOG/EORTC (*Radiation Therapy Oncology Group*/*European Organization for Research and Treatment of Cancer*) scale which defines the degrees of epithelitis in relation to the changes observed in erythema, dry or moist desquamation, pruritus, sweating, oedema, dermal ulceration, lesions with necrosis, hemorrhages, etc.

**Table 1 - Classification of the degree of epithelitis observed according to RTOG/EORTC scale**

| **DEGREES OF EPITHELITIS** | | |
|---|---|---|
| **DEGREES** | **Changes** | **Recommended supportive treatment** |
| **0** | Unchanged | Hydration 2 times/day |
| **I** | Erythema + dry desquamation + pruritus and Depilation + decreased sweating | Hydration + corticoids 2 times/day |
| **II** | Tense, shiny erythema + desquamation Patchy moist + moderate oedema | Hydration + corticosteroids 2 times/day + antibiotics |
| **III** | moist desquamation + ulceration + oedema confvea | Hydration + corticosteroids 2 times/day + Antibiotic + antifungal + healing agent |
| **IV** | Dermal ulceration + lesions with necrosis + non-trauma-induced hemorrhage | Moisturizing + corticosteroids 2 times/day + antibiotic + antifungal + wound healing +GIII dressings + cleansing + debriding |

In addition, data was collected on all parameters that could pose additional risks for the appearance of epithelitis, taking into account all previous circumstances or pathologies that could have a direct impact on the degree of toxicity of the treatment applied to the skin.

To complement the prospective study, a retrospective study was carried out to compare the results of patients treated with the composition of the invention with the results obtained from a review of 510 patients treated with the usual hydration used by the Nursing Service of the Hospital Clinico San Carlos in Madrid during the period from January 2012 to August 2013.

### RESULTS

### Prospective study

For the prospective study, 160 patients treated with radiotherapy between October 2013 and March 2014 were reviewed, of which 40 were in breast and 15 in head-neck location. They were randomly included in the treatment group with standard moisturizing creams (as described in the Material and Methods section) or in the group treated with the composition of the invention.

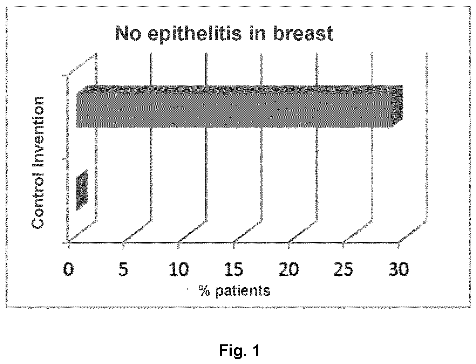

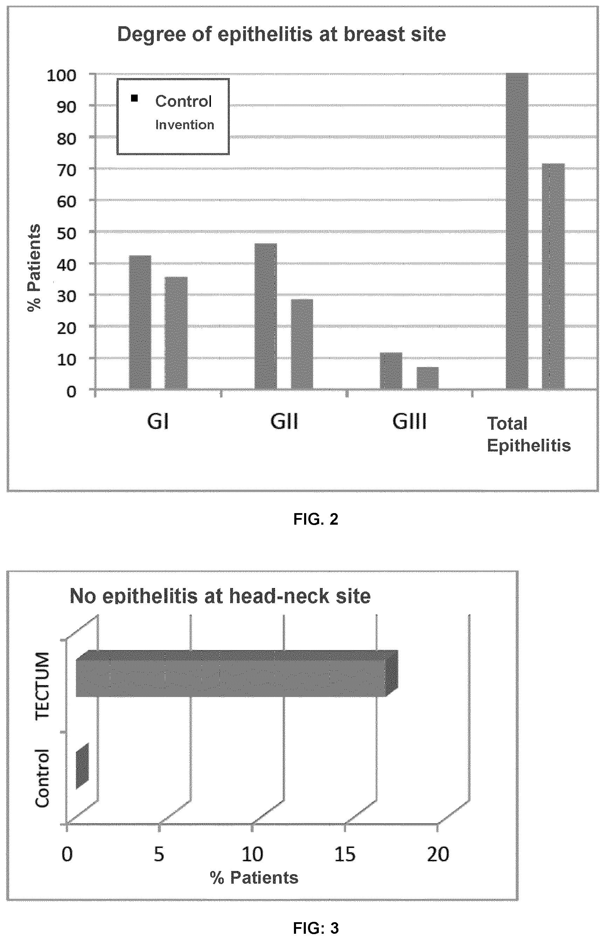

For breast localization, the comparative analysis was carried out with 26 patients in the treatment group with regular moisturizers and 13 in the treatment group with the composition of the invention. In the invention composition group 29 % of patients did not develop epithelitis, while in the control group all patients developed epithelitis to a greater or lesser degree (Figure 1). In addition, overall, the group with the composition of the invention had a lower incidence of GI, GII and Gill grades of epithelitis (Figure 2).

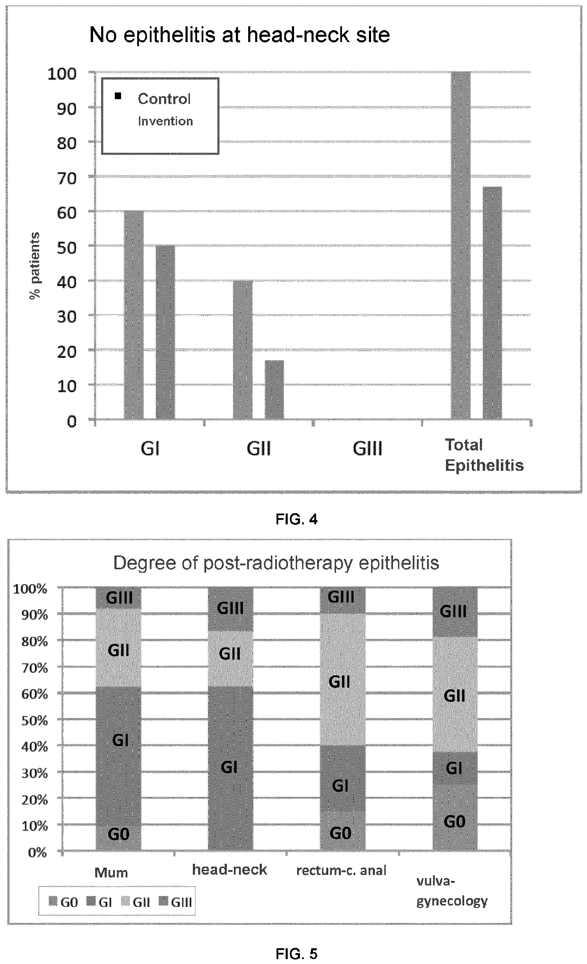

In the comparison of the group with moisturizing treatments versus the group treated with the composition of the invention in patients with head-neck cancer, 15 patients were analyzed, of which 10 were treated with regular moisturizing and five with the composition of the invention. In the group treated with the composition of the invention, 17% of patients did not develop epithelitis, while in the control group all patients developed epithelitis to a greater or lesser degree (Figure 3). Overall, grouping all epithelitis together, the group treated with the composition of the invention had a lower incidence of epithelitis (Figure 4).

### Retrospective study.

For the retrospective study, a review was made of 510 medical records of patients who received radiotherapy and who were treated with the usual hydration in the Nursing Service of the Hospital Clinico San Carlos in Madrid between January 2012 and August 2013.

These data provide valuable information on the degrees of epithelitis occurring during radiotherapy treatment depending on the irradiated field. Of the 510 medical records reviewed, 169 correspond to locations in: breast (109), head-neck (24), rectum/anal canal (20) ovulva/gynecological (16). The remaining 341 patients correspond to radiotherapy treatments in other locations.

It is observed that in both breast cancer and head-neck cancer, GI epithelitis predominates, affecting around 60% of patients. It is also relevant that only 9% of breast patients and none of the head-neck patients were free of some type of epithelitis (G0).

The other locations analyzed, with tumors in the rectum - anal canal or vulva - gynecological regions, show a lower incidence of epithelitis with a higher percentage of epithelitis-free patients. However, in patients where epithelitis starts, it is more severe with a predominance of Grades II and III (Figure 5).

To compare the retrospective study with the data obtained in patients treated with the composition of the invention, only patients with breast (109 patients) and head-neck (24 patients) localization were used.

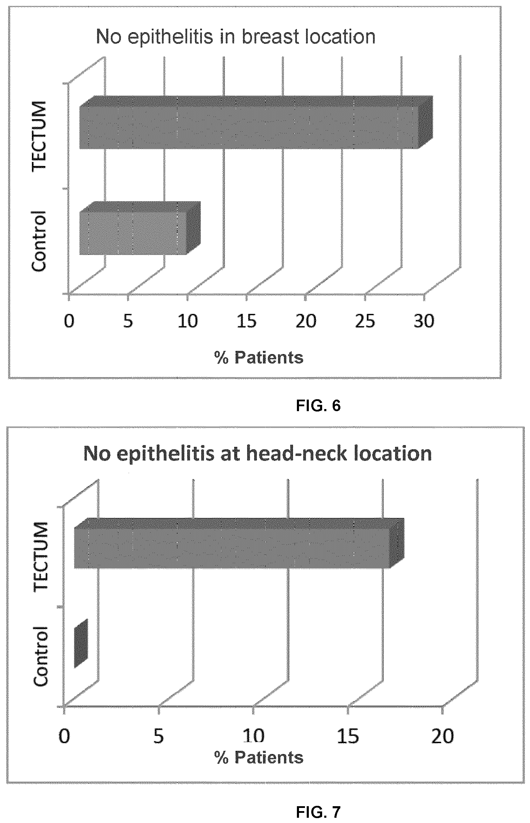

In the breast site, 9% of patients in the control group had G0; although this percentage is still lower than the 29% of patients who did not develop epithelitis in the group treated with the composition of the invention (Figure 6).

In the case of head-neck location, the results in the retrospective comparison were percentage-wise the same as those observed in the prospective study. None of the patients in the control group remained without epithelitis after radiotherapeutic treatments, whereas in the group treated with the composition of the invention we found 17% of patients with G0 (Figure 7).

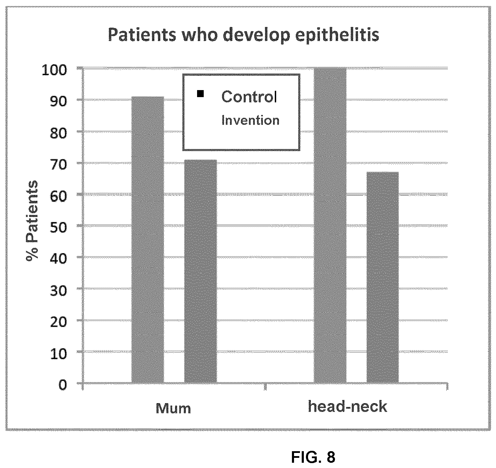

Another difference observed between the usual hydration control group and the group treated with the composition of the invention is that overall the percentage of patients with some type of radiodermatitis is lower in the group treated with the composition of the invention in both locations (breast and head-neck) (Figure 8).

It should be noted that the results on percentage of patients who did not develop epithelitis and percentage of patients who achieved GI, GII and Gill epithelitis are similar to those found in the prospective study.

### Case study

The following is a follow-up illustration of a particularly complicated case which, due to its special characteristics, was not included in the comparative studies described, but was treated with the composition of the invention. This is a 49-year-old patient with a head-neck radiation field and several associated risk conditions (concomitant treatment with monoclonal antibody, psoriasis, liver disease, diabetes, metallic heart valve and treatment with Sintrom). In this case, epithelitis evolved to Gill and GIV in the areas where two factors were superimposed (punctiform reaction border in the face and cheek area and exceeding 40 Gy total accumulated). However, in the neck-back area where the punctiform reaction was less intense and both risk factors did not overlap, it remained in GI.

The findings of this patient's follow-up are described below:
- At the start of radiotherapy treatment, no skin toxicity is observed. An increase in dryness appears after 16 Gy which is corrected only by adding a moisturizing booster with the composition of the facial invention in the nurse's office, after daily treatment.
- 05 February 2014. Radiotherapy received: 17 sessions with 34 Gy accumulated. GI epithelitis. Treatment: the composition of the invention Corporal 2 times a day, corticosteroids are not indicated due to her underlying pathology.
- 10 February 2014. Radiotherapy received: 20 sessions with 40 Gy accumulated. Epithelitis GII-III face neck and GI in the rest, back and sternal area. Treatment: the composition of the invention Corporal 2 times a day and cures are started 2 times a day.
- 12 February 2014. Radiotherapy received: 21 sessions with 42 Gy accumulated. Bleeding GIII-GIV epithelitis on face and neck, rest of GI. Treatment: the composition of the invention Corporal 2 times a day and cures every 12 hours. A stoppage is performed because the patient presents coagulation alterations with an INR (international normalized ratio) greater than 6.
- 17 February 2014. Radiotherapy received: 23 sessions with 46 Gy accumulated. GI epithelitis with hyperpigmentation on the back and neck; Gill epithelitis on both cheeks and ear lobes. Treatment: the composition of the invention twice a day and healing cream with hyaluronic acid and argentic sulphadiazine twice a day. A technical stop of the accelerator is made.
- 19 February 2014. Radiotherapy received: 23 sessions with 46 Gy accumulated. The INR has been corrected. Remission of epithelitis in cheeks and earlobes to GII and in neck and back GI with hyperpigmentation. Treatment: continued with the composition of the inventionCorporal 2 times a day and healing cream with hyaluronic acid and argentic sulphadiazine 1 time a day.
- 26 February 2014. Radiotherapy received: 28 sessions with 56 Gy accumulated. G0-GI epithelitis and hyperpigmentation on the back and neck; on the sides of the cheeks and anterior face of the neck and lobes of the right ear Gil with hardly any bleeding and with new epithelialised skin in the treated area. Treatment: the composition of the inventionCorporal 2-3 times a day and healing cream with hyaluronic acid and argentic sulfadiazine 2 times a day.
- 4 March 2014. Radiotherapy received: 32 sessions with 64 Gy accumulated. Back and front of neck with G0 epithelitis and hyperpigmentation. On the cheeks and earlobes GI epithelitis and in very isolated points GII without bleeding. Treatment: a reinforcement is made with moisturizing and the composition of the Facial invention once a day. The GI area presents erythema and dry skin without itching that does not require corticoids.
- 7 March 2014. End of radiotherapy. Radiotherapy received 35 sessions with 70 Gy accumulated. On the back, sternal area and anterior face of the neck the G0 epithelitis has subsided. On the cheeks, earlobes and lateral areas of the neck there is GI epithelitis (without itching) and new epithelitis in the treated area. There are isolated, open GII spots with discrete bleeding in the right hemi collar. Treatment: the composition of the invention Corporal 3 times a day and healing cream with hyaluronic acid and argentic sulphadiazine in the hemi collar and more labile areas of the neck are maintained.
- 13 March 2014. Post Treatment Review: Epithelitis G0 except in neck folds with GI. Treatment: the composition of the invention Corporal 2 times a day and cures 2 times a day.
- 20 March 2014. Post Treatment Review: Complete remission of Epithelitis (G0) in the entire treatment field. Treatment: the composition of the invention Corporal 2 times a day. Cures are no longer necessary.

### DISCUSSION

The tissue irradiation that forms part of the therapy for many cancers is not free of toxic effects, including the appearance of skin lesions. The nursing consultation is one of the most powerful tools for controlling the appearance of these side effects, minimizing their extent and treating any complications that may arise. The application of new treatments and their monitoring by the nursing services is important to make progress in the prevention and treatment of radiodermatitis.

The aim of the present study was to evaluate the influence of the application of the composition of the invention in a group of 20 patients (14 with breast and 6 with head-neck location) subjected to ionising radiation through a prospective and retrospective analysis where prospective data of 56 patients (40 with breast and 16 with head-neck location) and retrospective data of 133 patients (109 with breast and 24 with head-neck location) were reviewed respectively.

The analysis of the data shows several differences between the results obtained in the group of patients treated with the composition of the invention versus those observed in the group of patients treated with standard hydration (Gold standard), despite the fact that both groups had similar characteristics of age and dose of radiotherapy received.

In the prospective analysis, the group that received the composition of the invention presented 29 % of patients who did not develop epithelitis, while in the usual hydration group all patients developed epithelitis to a greater or lesser degree (Figure 1) and at an overall level the group of the composition of the invention presented a lower incidence of GI, GII and Gill degrees of epithelitis (Figure 2).

This same pattern was observed in the retrospective analysis of patients where the group treated with the composition of the invention presented again a higher percentage of patients without epithelitis after radiation (G0) compared to the group that had a treatment with usual hydration (Figure 6 and 7).

If the fractions are 2.66 Gy/day, grade I epithelitis appears earlier in all the groups of patients analysed and also, although to a lesser extent, in the group treated with the composition of the invention, where it is observed from a cumulative dose of 16 Gy.

It is noteworthy that in patients treated with the composition of the invention who had no other added risks and irradiated with fractions of 2 Gy/day, grade I epithelitis appears in the range of 25-30 Gy accumulated on average. This result is clearly different from that found in the review shown in table 1 (3), where we observed that early transient erythema appears from 2 Gy, mild erythema and depilation between 6-10 Gy and definite erythema and hyperpigmentation from 12 Gy onwards.

In the specific analysis in response to more conventional treatments and with localization in the breast, especially in lesions in the sub-mammary fold, we observed that some points appear with loss of continuity of the skin, but in the cases treated with the composition of the invention, when these dead skins are removed and cleaned, the new layer that appears is integrated and therefore cannot be considered a wound (there is new epithelialization) and the patient does not report the typical symptoms of stinging on contact due to open lesion.

Except in head and neck cases, where concomitant Cetuximab-type treatments are also added, in patients who were exclusively administered the composition of the invention, grade I epithelitis remained stable during the first week after the end of radiotherapy treatment, reinforced with topical corticosteroid treatments.

In breast patients where isolated grade II epithelitis spots appeared in the sulcus or axilla, remission occurred rapidly with treatment with the composition of the invention and hygiene care with hyaluronic acid ointment associated with sulphadiazine or in the case of affected mucosa we used Nitrofural.

### CONCLUSIONS

None of the treatment groups with regular hydration presented any case without epithelitis (G0). In the group of the composition of the invention in the Head Neck location treatments we found 17% of patients who did not have any type of epithelitis (G0) and when the location was Breast there were 29% of patients who did not develop epithelitis, globally all the patients presented a lower incidence of grades (GI, GII and Gill) (Figures 1, 2, 6 and 7).

Patients who were treated in the group of the composition of the invention and who ended up with GI grades and hyperpigmentation achieved complete remission after one week or maximum thirteen days post-treatment in all cases studied.

In all cases and/or locations where some degree of toxicity was present, epithelialisation until complete remission is notably shortened in the group of patients treated with the composition of the invention compared to the usual treatments, which results in a reduction of complications such as infections and also reduces the cost and time of treatment, and above all, there is a benefit in the patient's quality of life, as discomfort due to displacement is avoided and symptoms such as pain are reduced.

**CLAIMS**

## Claims

1. A peptide **characterized in that** it comprises SEQ ID sequence NO: 1, for use in the treatment and/or prevention of radiodermatitis in a subject, or in the regeneration of tissue following the application of radiotherapy to a subject.

2. A peptide for use according to claim 1, **characterized in that** the peptide comprises the amino acid sequence SEQ ID NO: 2, SEQ ID NO: 3, SEQ ID NO: 4, SEQ ID NO: 5 or SEQ ID NO: 6.

3. A peptide for use according to claim 1 or 2, wherein the peptide comprises at least one nuclear localization sequence attached to at least one of the carboxyl or amino terminal end of its amino acid sequence, preferably, the nuclear localization sequence is attached to the carboxyl terminal end of the peptide.

4. The peptide for use according to claim 3, wherein the nuclear localization sequence comprises the amino acid sequence SEQ ID NO: 7 and/or SEQ ID NO: 8.

5. A polynucleotide encoding a peptide according to any one of claims 1 to 4, for use in the treatment and/or prevention of radiodermitis in a subject, or in tissue regeneration following the delivery of radiotherapy to a subject.

6. A polynucleotide for use according to claim 5, wherein the polynucleotide comprises the sequence SEQ ID NO: 17, SEQ ID NO: 18, SEQ ID NO: 19, SEQ ID NO: 20, SEQ ID NO: 21 or SEQ ID NO: 22.

7. A vector comprising a polynucleotide according to claim 5 or 6, for use in the treatment and/or prevention of radiodermitis in a subject, or in tissue regeneration following the delivery of radiotherapy to a subject.

8. A cell comprising a peptide according to any one of claims 1 to 4, a polynucleotide according to claim 5 or 6, or a vector according to claim 7, for use in the treatment and/or prevention of radiodermitis in a subject, or in tissue regeneration following the application of radiotherapy to a subject.

9. A cell for use according to claim 8, wherein the cell is a prokaryotic or eukaryotic cell, preferably, the eukaryotic cell is a mammalian cell, more preferably, the mammalian cell is a human cell.

10. A nanoparticle comprising a peptide according to any one of claims 1 to 4, a polynucleotide according to claim 5 or 6, a vector according to claim 7, or a cell according to claim 8 or 9, for use in the treatment and/or prevention of radiodermitis in a subject, or in tissue regeneration following the application of radiotherapy to a subject.

11. A nanoparticle for use according to claim 10, wherein the nanoparticle is a nanosphere, a nanocapsule, a micelle, a liposome or a dendrimer.

12. A composition comprising a peptide according to any one of claims 1 to 4, a polynucleotide according to claim 5 or 6, a vector according to claim 7, a cell according to claim 8 or 9, or a nanoparticle according to claim 10 or 11, for use in the treatment and/or prevention of radiodermitis in a subject, or in tissue regeneration following the application of radiotherapy to a subject.

13. A composition for use according to claim 12, wherein the composition is formulated for topical application to the subject.

14. Kit comprising a peptide according to any one of claims 1 to 4, a polynucleotide according to claim 5 or 6, a vector according to claim 7, a cell according to claim 8 or 9, a nanoparticle according to claim 10 or 11, or composition according to claim 12 or 13, for use in the treatment and/or prevention of radiodermitis in a subject, or in tissue regeneration following the application of radiotherapy to a subject.

15. Peptide, polynucleotide, vector, cell, nanoparticle, composition or kit for use according to any one of claims 1 to 14, wherein the radiodermatitis comprises the occurrence of skin desquamation, skin erosions, skin and mucous membrane irritations, and/or skin and mucous membrane dehydration.
